# FASCICULE DE BREVET EUROPEEN

(11) **EP 1 481 708 B1**
(45) Date de publication et mention de la délivrance du brevet: **05.03.2008**
(21) Numéro de dépôt: 04291335.0
(22) Date de dépôt: 27.05.2004
(51) Int. Cl.: A61N 1/372

(54) **Système de collecteur d'onde pour la réception de signaux d'induction magnétique émis par un appareil médical actif implanté**
Wellenempfangsvorrichtung zum Empfang von aus einem implantierbaren aktiven Gerät ausgesendeten elektromagnetischen Signalen
Wave receiver system for receiving electromagnetic signals emitted by an implantable active device

(30) Priorité: 28.05.2003 FR 0306461
(43) Date de publication de la demande: 01.12.2004
(73) Titulaire: ELA MEDICAL, 92541 Montrouge (FR)
(72) Inventeur: Wanderstok, Georges, 92140 Clamart (FR); Tanche, Vincent, 91940 Gometz le Chatel (FR)
(74) Mandataire: Dupuis-Latour, Dominique

(56) Documents cités:
- EP-A- 0 797 317
- WO-A-01/05467

## Description

La présente invention concerne le domaine des dispositifs médicaux implantés actifs, et plus particulièrement le recueil des signaux émis lors de séquences de communication entre le dispositif implanté et une console de contrôle extérieure.

Les dispositifs médicaux implantés actifs comprennent notamment les stimulateurs cardiaques, défibrillateurs, cardioverteurs et dispositifs multisite, les appareils neurologiques, les pompes de diffusion de substance médicale, et les implants cochléaires.

Ces appareils, une fois implantés, sont programmés de l'extérieur au moyen d'une console appelée "programmateur". La vérification des paramètres de l'implant ou la transmission d'informations enregistrées par celui-ci s'effectuent par voie électromagnétique, appelée "télémétrie" dans la technique en question. Cette console est munie d'un organe récepteur qui est placé en regard du site de l'implant. Cet organe, souvent désigné "antenne", comprend une bobine qui capte le champ magnétique en provenance de l'appareil implanté.

Une configuration couramment employée consiste à employer une antenne unique, placée dans la semelle de la tête du programmateur. Le signal induit est amplifié et traité pour extraire l'information issue de l'implant.

Cette configuration présente cependant l'inconvénient de recevoir à la fois le signal utile et toutes les perturbations radioélectriques environnantes, d'autant plus difficiles à éliminer que la bande passante du système doit être large.

Pour augmenter le rapport signal/bruit, le EP-A-0 661 077 (ELA Médical) propose d'utiliser une pluralité de collecteurs d'onde et d'opérer sur les signaux captés une combinaison linéaire particulière permettant essentiellement de conserver la composante utile du signal en éliminant la majeure partie des composantes de bruit en provenance de sources parasites.

L'amélioration du rapport signal/bruit est en effet essentielle si l'on souhaite augmenter le débit de transmission des données de l'implant vers le programmateur, par exemple si l'on veut télécharger des données volumineuses stockées dans l'implant telles que des enregistrements Holter d'ECG endocavitaires sur une durée de plusieurs heures, représentant un volume de données de plusieurs mégabits.

Pour améliorer encore les performances du programmateur, le EP-A-0 797 317 (ELA Médical) propose d'utiliser une géométrie particulière de collecteurs d'ondes permettant de recueillir des signaux pour lesquels la composante de bruit aura déjà été fortement réduite dès le recueil, avant même le traitement par les circuits électroniques. Ce document propose de disposer deux bobines distinctes sur un circuit magnétique commun tel qu'une coupelle en ferrite, à savoir une bobine de réception et une bobine de compensation. La bobine de réception est par exemple bobinée sur le noyau central de la coupelle ferrite, tandis que la bobine de compensation est bobinée coaxiale sur la périphérie de la coupelle. De cette manière, les lignes de champ de l'induction magnétique de la composante utile traversent une seule fois la bobine réceptrice, alors qu'elles traversent deux fois, et en sens opposé, la bobine de compensation. En revanche, les inductions parasites lointaines traversent dans le même sens et presque identiquement les deux bobines, ce qui permet aisément d'isoler ces signaux parasites pour les soustraire du signal utile.

Le WO-A-01/05467 (Medtronic) propose par ailleurs d'utiliser pour la réception deux antennes distinctes, mais disposées de manière concentrique et coplanaire. Les signaux issus des deux enroulements sont soustraits de manière à éliminer les perturbations lointaines, qui induisent des signaux identiques mais en opposition de phase. Le signal émis à faible distance par l'implant induit des tensions de phases opposées mais d'amplitudes différentes, qui ne s'annulent donc pas et permettent d'extraire du bruit environnant un signal utile dont le traitement ultérieur peut être simplifié.

Avec ces configurations connues mettant en oeuvre une pluralité de bobines, la différenciation est excellente, mais au détriment de la portée pratique. En effet, la distance entre les antennes étant limitée par les dimensions de la tête du programmateur, un implant situé à plus de quelques centimètres des antennes est déjà considéré comme une perturbation lointaine ; l'atténuation du signal en fonction de la distance tête-implant s'accroît alors beaucoup plus vite que la loi classique d'atténuation en fonction de l'inverse du carré de la distance.

Pour pallier cet inconvénient, on peut envisager de disposer les deux antennes côte à côte. Avec cette configuration, une "zone morte" apparaît cependant dans une bande située entre les antennes, formant un "puits différentiel" où aucun signal ne peut être reçu. Cette bande empiète sur les surfaces de capture des antennes et réduit d'autant l'aire de réception utile de la tête. D'autre part, la réjection des signaux dans la zone morte varie très rapidement d'un point de vue spatial, de sorte qu'un petit mouvement latéral de la tête par rapport à l'implant peut engendrer une variation d'amplitude très importante, voire une inversion de la phase du signal utile.

Pour remédier à cet inconvénient et aux inconvénients précités des systèmes connus, l'invention propose une nouvelle géométrie particulière des collecteurs d'onde, permettant de maximiser l'aire dans laquelle peut s'établir la communication avec l'implant.

La partie caractérisante de la revendication 1 de la présente invention reprend les termes décrivant cette nouvelle géométrie particulière des collecteurs d'onde.

Comme on le verra par la suite, la configuration selon l'invention présente les avantages suivants :
- par rapport à une antenne différentielle à coupelle ferrite (telle que décrite dans le EP-A-0 797 317 précité) : la suppression de la ferrite procure des avantages en terme de volume du cône de détection, prix, poids, solidité, facilité d'intégration, fiabilité et pérennité ;
- par rapport à une construction coaxiale ou concentrique (telle que décrite par exemple dans le WO-A-01/05467 précité) : absence de réduction de portée des signaux proches, en conservant une bonne réjection des perturbations lointaines ;
- par rapport à une construction coplanaire symétrique : réduction de l'aire du puits différentiel ;
- possibilité d'utiliser un seul canal d'amplification, la différence entre les signaux de réception et de compensation pouvant s'effectuer par une simple mise en série et en opposition de phase des enroulements.

À cet effet, l'invention propose un dispositif du type décrit par le EP-A-0 661 077 précité pour la réception de signaux émis par un appareil médical actif implanté, c'est-à-dire comportant des moyens collecteurs d'onde pour la réception d'une induction magnétique comprenant une composante utile émise par l'appareil implanté et une composante parasite d'origine externe, ces moyens collecteurs d'onde comportant au moins une bobine de réception et au moins une bobine de compensation.

Selon l'invention, la bobine de réception et la bobine de compensation comprennent chacune au moins une série de spires enroulées autour de mandrins respectifs, en matériau de préférence non magnétique, ces mandrins étant disposés côte à côte avec leurs axes respectifs distants et parallèles et leurs sections n'étant pas concourantes, et la section du mandrin de la bobine de compensation étant inférieure à celle du mandrin de la bobine de réception.

Très avantageusement, la série de spires de la bobine de compensation est fractionnée en une pluralité de groupes de spires enroulés sur un même mandrin avec espacement axial et reliés en série.

Les spires de la bobine de compensation peuvent également être enroulées en nid d'abeilles.

Dans une forme de réalisation avantageuse, la série de spires de la bobine de compensation est fractionnée en une pluralité de groupes de spires enroulés sur des mandrins distincts et reliés en série, les différents mandrins étant d'axes parallèles et situés à même distance de la bobine de réception.

Lorsque la série de spires de la bobine de compensation est fractionnée, les différents groupes de spires présentent de préférence des sections d'enroulement et/ou des nombres de spires différents.

On va maintenant décrire un exemple de mise en oeuvre du dispositif de l'invention, en référence aux dessins annexés.
La figure 1 est une vue en élévation du système d'antenne selon l'invention, avec une antenne d'émission/réception associée à une antenne de compensation.
La figure 2 est une vue en plan d'un système d'antenne montrant une bobine d'émission/réception associée à une pluralité d'antennes de compensation.

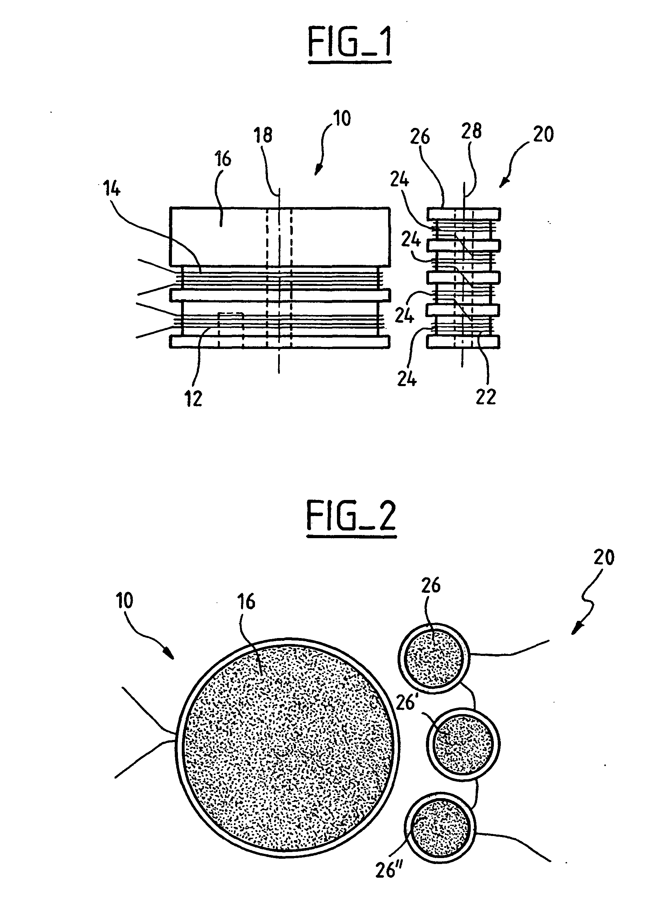

Sur les figures 1 et 2, on a représenté la structure du système de collecteurs d'onde d'un programmateur selon l'invention.

On sait que, dans le cas de la télémétrie des informations en provenance d'un implant, les signaux utiles aussi bien que les signaux parasites sont véhiculés sous la forme de champs magnétiques, le signal total reçu par un collecteur d'onde se décomposant en un signal utile et un signal parasite.

Comme cela est exposé dans le EP-A-0 661 077 précité, il est possible d'extraire du signal total la composante de signal utile par une combinaison linéaire particulière des signaux délivrés par une pluralité de bobines distinctes recueillant chacune des flux magnétiques induits propres et produisant des tensions correspondantes appliquées à des amplificateurs respectifs dont les sorties sont combinées entre elles dans un étage sommateur.

La présente invention propose une géométrie particulière pour ces bobines, illustrée figures 1 et 2.

Le système de collecteur d'ondes comprend au moins deux bobines, comme dans le mode de réalisation illustré figure 1, à savoir une bobine d'émission/réception 10 destinée essentiellement, dans sa fonction réception, à recueillir le signal utile, et une ou plusieurs bobines dites "de compensation" 20 destinées à recueillir essentiellement une composante de bruit.

L'antenne d'émission/réception 10 comprend un bobinage de réception 12 et un bobinage d'émission 14 enroulés ensemble autour d'un mandrin commun 16. Ce mandrin est de préférence un mandrin à air (dépourvu de matériau magnétique tel qu'un ferrite), par exemple de forme cylindrique d'axe 18, de la plus grande surface compatible avec les dimensions physiques de la tête du programmateur. L'aire dans laquelle la communication avec l'implant peut s'établir est ainsi maximale.

L'antenne de compensation 20 comprend un bobinage 22 enroulé autour d'un mandrin 26, qui est également de préférence un mandrin à air mais de diamètre beaucoup plus faible que le mandrin 16 de l'antenne d'émission/réception. Le mandrin 26 est disposé collatéralement au mandrin 16, les axes respectifs 28, 18 de ces mandrins étant parallèles. L'enroulement de réception 12 et l'enroulement de compensation 22 devant avoir la même sensibilité vis-à-vis des perturbations lointaines, leur nombre de spires doit être inversement proportionnel à leur surface.

On remarquera que l'aire de réception de l'antenne de compensation 20 étant beaucoup plus réduite que celle de l'antenne d'émission/réception 10, la zone de puits différentiel située entre les deux antennes est limitée.

L'enroulement 22 de l'antenne de compensation 20 est avantageusement réparti en plusieurs groupes de spires successifs 24.

En effet, l'antenne de compensation ne doit pas dépasser un nombre de spires critique, correspondant à une inductance maximale limitant la bande passante du système.

Pour cela, dans une première forme de réalisation illustrée figure 1, l'enroulement 22 est réalisé en plusieurs groupes 24 de spires rangées, coaxiaux mais disposés à distance les uns les autres sur le même mandrin 26. Avantageusement, les nombres de spires des différents groupes 24 ne sont pas identiques, de manière que chaque groupe ait une fréquence de résonance propre différente, conduisant à un pic de résonance global moins marqué pour l'antenne de compensation 20.

En variante l'enroulement 22 de l'antenne de compensation peut être réalisé par la technique dite du nid d'abeille,c'est-à-dire avec des spires mises en treillis aéré, le moins jointivement possible, en forçant un chevauchement ordonné.

Dans une autre forme de réalisation, utilisable en variante ou en complément et illustrée figure 2, les différents groupes de spires de l'antenne de compensation 20 sont répartis sur autant de mandrins différents 26, 26', 26", pour donner des bobinages, non couplés entre eux, connectés en série. Les différents groupes de spires sont disposés à faible distance (quelques centimètres) de l'antenne de réception, pour éviter de retomber sur l'inconvénient exposé plus haut des antennes coplanaires de surface égale.

L'inductance globale de l'antenne de compensation est fortement réduite et ses performances en termes de bande passante sont accrues. Ainsi, dans l'exemple d'une antenne de compensation fractionnée en trois sections d'inductance L1, L2 et L3 de couplage mutuel négligeable, si l'inductance d'une antenne unique non fractionnée (comme illustré figure 1) vaut L, celle de L1, L2 ou L3 sera L/3² = L/9 dans le cas d'une antenne fractionnée (comme illustré figure 2). Les trois fractions ont donc ensemble une inductance totale L' = L1 + L2 + L3 = 3.L/9 = L/3 : la bande passante est ainsi triplée pour une charge résistive donnée.

On notera enfin qu'il est possible d'utiliser pour la réception des circuits simplifiés ne comportant qu'un seul canal d'amplification, la différence entre les signaux de réception et de compensation, c'est-à-dire avec soustraction des signaux parasites du signal utile, pouvant s'effectuer par une simple mise en série et en opposition de phase des enroulements de l'antenne de réception et de l'antenne de compensation.

## Revendications

1. Un dispositif de réception de signaux émis par un appareil médical actif implanté, ce dispositif comportant des moyens collecteurs d'onde pour la réception d'une induction magnétique comprenant une composante utile émise par l'appareil implanté et une composante parasite d'origine externe, ces moyens collecteurs d'onde comportant au moins une bobine de réception (10) et au moins une bobine de compensation (20),
**caractérisé en ce que** la bobine de réception (10) et la bobine de compensation (20) comprennent chacune au moins une série de spires (12, 22) enroulées autour de mandrins respectifs (16, 26), de préférence en matériau non magnétique, ces mandrins étant disposés côte à côte avec leurs axes respectifs (18, 28) distants et parallèles et leurs sections n'étant pas concourantes, et la section du mandrin (26) de la bobine de compensation (20) étant inférieure à celle du mandrin (16) de la bobine de réception (10).

2. Le dispositif de la revendication 1, dans lequel la série de spires (22) de la bobine de compensation (20) est fractionnée en une pluralité de groupes de spires (24) enroulés sur un même mandrin (26) avec espacement axial et reliés en série.

3. Le dispositif de la revendication 1, dans lequel les spires de la bobine de compensation sont enroulées en nid d'abeilles.

4. Le dispositif de la revendication 1, dans lequel la série de spires (22) de la bobine de compensation (20) est fractionnée en une pluralité de groupes de spires enroulés sur des mandrins distincts (26, 26', 26") et reliés en série, les différents mandrins étant d'axes parallèles et situés à même distance de la bobine de réception.

5. Le dispositif de la revendication 2 ou 4, dans lequel les différents groupes de spires de la bobine de compensation présentent des sections d'enroulement et/ou des nombres de spires différents.

## Claims

1. A device for receiving signals transmitted by an active implanted medical apparatus, said device comprising wave collecting means for the reception of a magnetic induction including a useful component transmitted by the implanted apparatus and a parasitic component of external origin, said wave collecting means comprising at least one reception coil (10) and at least one compensation coil (20),
**characterised in that** the reception coil (10) and the compensation coil (20) each comprises at least one series of turns (12, 22) wound on respective mandrels (16, 26), preferably made of a nonmagnetic material, said mandrels being positioned side by side with their respective axes (18, 28) spaced apart and parallel and their cross-sectional areas not being superimposed, and the cross-sectional area of the mandrel (26) of the compensation coil (20) being smaller than the cross-sectional area of the mandrel (16) of the reception coil (10).

2. The device of claim 1, wherein the series of turns (22) of the compensation coil (20) is split into a plurality of groups of turns (24) wound on the same mandrel (26), axially spaced apart and connected in series.

3. The device of claim 1, wherein the turns of the compensation coil are wound in a honeycomb pattern.

4. The device of claim 1, wherein the series of turns (22) of the compensation coil (20) is split into a plurality of groups of turns wound on separate mandrels (26, 26', 26") connected in series, the separate mandrels having their axes in parallel and being located at the same distance from the reception coil.

5. The device of claim 2 or 4, wherein the different groups of turns of the compensation coil have a different number of turns have different winding cross-sectional areas and or different numbers of turns.

## Patentansprüche

1. Eine Vorrichtung zum Empfangen von Signalen, welche von einem implantierten aktiven medizinischen Gerät ausgesandt werden, wobei diese Vorrichtung Wellenempfangsmittel zum Empfang einer magnetischen Induktion, welche ein zweckmäßiges Bestandteil, welche von dem implantierten Gerät ausgesendet wird, und einen parasitären Bestandteil aus externer Herkunft enthält, einschließt, wobei diese Wellenempfangsmittel mindestens eine Empfangsspule (10) und mindestens eine Ausgleichsspule (20) umfassen,
**dadurch gekennzeichnet, dass** die Empfangsspule (10) und die Ausgleichsspule (20) zumindest jede eine Reihe von Wicklungen (12, 22), welche um jeweilige Drehspindeln (16, 26), vorzugsweise aus einem nichtmagnetischen Material, gerollt sind, enthält, wobei diese Drehspindeln Seite an Seite mit ihren beabstandeten und parallelen jeweiligen Achsen (18, 28) angeordnet sind und ihre Querschnitte nicht konvergent sind, und wobei der Querschnitt der Drehspindel (26) der Ausgleichsspule (20) geringer als jener der Drehspindel (16) der Empfangsspule (10) ist.

2. Die Vorrichtung des Anspruchs 1, in welcher die Reihe der Bindungen (22) der Ausgleichsspule (20) in eine Vielzahl von Bindungsgruppierungen (24) geteilt ist, welche auf einer selbigen Drehspindel (26) mit axialem Abstand und in Serie verbunden aufgerollt sind.

3. Die Vorrichtung aus Anspruch 1, in welcher die Bindungen der Ausgleichsspule in einem Bienennest aufgerollt sind.

4. Die Vorrichtung des Anspruchs 1, in welcher die Reihe der Bindungen (22) der Ausgleichsspule (20) in eine Vielzahl von Windungsgruppierungen aufgeteilt ist, welche auf jeweiligen Drehspindeln (26, 26', 26") aufgerollt sind und welche in Serie verbunden sind, wobei die unterschiedlichen Drehspindeln parallelachsig und in gleichem Abstand von der Empfangsspule angeordnet sind.

5. Die Vorrichtung der Ansprüche 2 oder 4, in welcher die verschiedenen Windungsgruppierungen der Ausgleichsspule verschiedene Aufrollquerschnitte und/oder Anzahlen von Windungen aufweisen.
